# EUROPEAN PATENT SPECIFICATION

(11) **EP 1 521 567 B1**
(45) Date of publication and mention of the grant of the patent: **13.12.2006**
(21) Application number: 03763681.8
(22) Date of filing: 30.06.2003
(51) Int. Cl.: A61K 8/49, A61K 8/73, A61K 8/92, A61Q 5/00, A61Q 5/02

(54) **HAIR AND/OR SCALP TREATMENT COMPOSITION**
ZUSAMMENSETZUNG FÜR DIE BEHANDLUNG VON HAAR- UND/ODER KOPFHAUT
COMPOSITION POUR LE TRAITEMENT DES CHEVEUX ET/OU DU CUIR CHEVELU

(30) Priority: 15.07.2002 EP 02254955
(43) Date of publication of application: 13.04.2005
(73) Proprietor: UNILEVER PLC, London EC4P 4BQ (GB); UNILEVER N.V., 3013 AL Rotterdam (NL)
(72) Inventor: CHANDRA, Lalitesh, Unilever R & D Port Sunlight, Wirral, Merseyside CH63 3JW (GB)
(74) Representative: Tansley, Sally Elizabeth
(86) International application number: PCT/EP2003/006991
(87) International publication number: WO 2004/006876

(56) References cited:
- WO-A-01/47481
- WO-A-87/06827
- US-B1- 6 323 166
- DATABASE KOSMET [Online] JADHAV V A (PHARMACEUTICAL DIVISION, DEPARTMENT OF CHEMICAL TECHNOLOGY, MUMBAI UNIVERSITY, MUMBAI 400019, MAHARASHTRA, INDIA) ET A: "ANTIDANDRUFF HERBAL COSMECEUTICALS - A NOVEL APPROACH" retrieved from STN Database accession no. 24660 XP002219813 & IFSCC CONFERENCE, MAY 7 - 9, 2001, STOCKHOLM, SWEDEN, "QUALITY OF COSMETICS;AN EXPLOSIVE MYTH ?", P211-216, 11 REFS MEETING ORGANIZER: SCANCOS, IFSCC AVAILABILITY: SCANCOS, IFSCC,
- DATABASE CA [Online] CHEMICAL ABSTRACTS SERVICE, COLUMBUS, OHIO, US; CHENG, JINXUE: "Antiitching and antidandruff shampoo containing extracts of snake tissues and medicinal plants" retrieved from STN Database accession no. 131:9442 XP002219806 & CN 1 116 234 A (PEOP. REP. CHINA) 7 February 1996 (1996-02-07)

## Description

This invention relates to a hair and/or scalp treatment composition.

Dandruff is a condition experienced by many people worldwide. The dandruff condition varies from mild symptoms such as flaking skin and slight itchiness of the scalp to severe inflammation and itchiness of the scalp.

*Malassezia* yeasts, such as *Malassezia furfur,* are believed by some to be the main cause of dandruff and, whilst this might not represent the full scientific picture of the situation, *Malassezia* yeasts do appear to be closely associated with dandruff. Hence, the strategy conventionally used for the treatment of dandruff is the topical application of antifungals such as zinc pyrithione (ZnPTO), octopirox, climbazole and ketoconazole which are normally delivered from a shampoo. These antifungal agents remove (or at least reduce the level of) the *Malassezia* from the scalp, and provide moderately effective treatment of the dandruff condition. An example of such technology is described in WO 01/47481.

The conventionally used antimicrobial agents may be perceived by the user as being harsh on the skin and/or the hair. Hair treated with the compositions can, for example, sometimes be thought of as being in less good condition.

It is known that some naturally occurring oils can have antifungal and antimicrobial activity and these oils have been used, for example, as antifungal agents for treating plants and for treating certain skin conditions.

Database Kosmet no.24660 - describes tulsi oil and tea tree oil as possible antidandruff agents. US6323166 describes terpene esters and terpene hydrocarbons as having antibacterial effects. Phenols such as eugenol are described as having antibacterial effects by WO87/06827.

There remains a need for compositions for treating dandruff that have a higher activity than compositions and compounds that are conventionally used. Compositions having a higher activity allow the amount of active component to be reduced or provide a greater effect from the same weight of the component used. There also remains a need for antidandruff compositions that are perceived as being milder than the conventional compositions.

An oil formulation useful as a mouthwash, cream or shampoo is disclosed in AU-A-200124760. The oil comprises 0.25% to 15% weight/volume tea tree oil and 11% to 15% weight/volume surfactant.

KR-A-2001019410 discloses a shampoo composition for preventing dandruff containing a surfactant, oil and zinc pyrithione. The oil may be spice, tea tree oil, pine oil, milk oil, benzyl alcohol or liquid paraffin.

The present invention is based on the unexpected finding that certain compounds, at least some of which may be present in naturally occurring oils, exhibit a synergistic effect in combination with certain synthetic antimicrobial agents. This finding is especially useful in the treatment and/or prevention of dandruff.

According to the invention in a first aspect, there is provided a hair and/or scalp treatment composition comprising tulsi oil and a metal pyrithione, wherein the tulsi oil and the metal pyrithione are capable of exhibiting a synergistic antimicrobial activity.

The present invention is based on the surprising finding of a synergistic effect between synthetic antimicrobial agents and certain compounds. This synergistic effect provides a greater activity in treating dandruff and/or allows the amount of the antidandruff agent used in the compositions (such as the synthetic antimicrobial agent) to be reduced whilst providing the same antidandruff activity. Moreover, since the compounds that provide the synergistic effect may be present in natural oils, it is believed that they can have the benefits associated with the use of natural oils in the treatment of hair and/or skin, such as conditioning benefits for hair including, for example, smoothness, softness and shine.

Tulsi oil is obtained from the Holy Basil plant, Ocimum sanctum and is known to have beneficial antimicrobial and/or insecticidal properties. Tulsi oil is typically extracted from the leaves of the plant. Tulsi oil comprises, in addition to relatively high amounts of eugenol compared to other natural oils, other component compounds such as terpinen-4-ol, linalool and 4-allylanisole.

A method for the separation of tulsi oil from the leaves of the plant is described in Laskar et al, *Journal of the Indian Chemical Society,* 1988, vol 65, pages 301-302. This document also describes the major constituents of tulsi oil. Further constituents of the plant are disclosed in Noerr *et al, Planta Medica,* 1992, vol 58, page 574.

In the first aspect of the invention, tulsi oil is used together with a metal pyrithione.

In the third aspect of the invention, component compounds of tulsi oil may be employed together with the synthetic antimicrobial agent. The component compounds may be used as a single compound or a combination of two or more compounds. Typically, the component compounds will be used as substantially pure single compounds eg, comprising at least 90% by weight of the compound, more preferably at least 95% by weight, based on the total weight of the component compounds present.

Preferred compounds are terpinen-4-ol, linalool, eugenol, alpha-terpineol and mixtures thereof, including mixtures of two, three and all four of these compounds. Of these, eugenol is particularly preferred on account of the fact that it exhibits a synergistic antimicrobial effect against *M.furfur* with a wide range of different synthetic antimicrobial agents. The structures of the preferred compounds are given below:

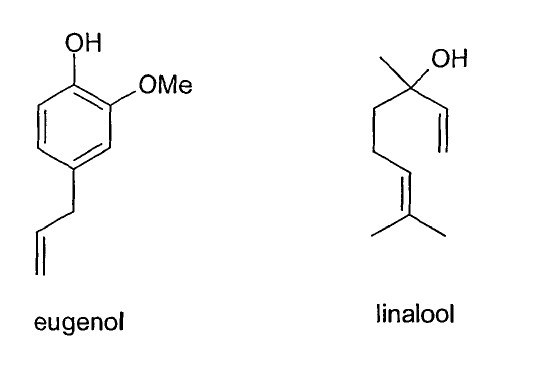

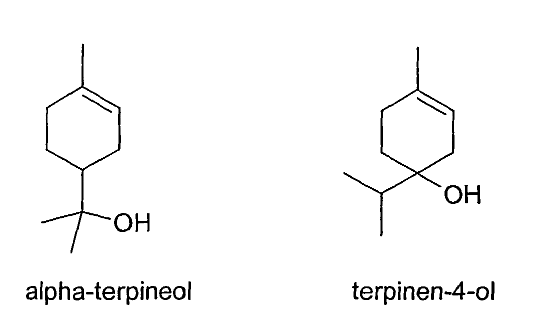

The tulsi oil or the compound (or mixture of compounds) is preferably present in a hair and/or scalp treatment composition in an amount of from 0.0001% to 5% by weight, more preferably from 0.001% to 3% by weight, even more preferably from 0.01% to 2% by weight, such as about 1% by weight, based on the total weight of the composition.

The synthetic antimicrobial agent (or mixture of such agents), such as for example zinc pyrithione, is preferably present in the composition in an amount of from 0.01% to 5% by weight, more preferably from 0.2% to 3% by weight.

The weight ratio of the tulsi oil or the compound (or mixture of compounds) to the synthetic antimicrobial agent (or mixture of such agents) that is used in the invention can vary widely, for example from 100:1 to 1:100, and preferably lies within the range of from 1:2 to 1:50 for the most effective antidandruff effect.

In the first aspect of the invention, the metal pyrithione is preferably zinc pyrithione. The metal pyrithione will usually be present in compositions of the invention in solid particulate form.

The zinc pyrithione may be used in any particle form including, for example, crystalline forms such as platelets and needles and amorphous, regularly or irregularly shaped particles. If zinc pyrithione is present in a composition, a suspending agent is preferably used to prevent or inhibit the settling of the particles out of the composition. The average particle diameter of the zinc pyrithione particles (ie, their maximum dimension) is typically from about 0.2 to about 50 µm, preferably from about 0.4 to about 10 µm.

Synthetic antimicrobial agents typically display a minimum inhibitory concentration of about 50 mg/ml or less against *Malassezia furfur.*

Combinations of synthetic antimicrobial agents and compounds that exhibit a synergistic microbial activity against *M.furfur* and which can therefore be expected to exhibit a synergistic antidandruff effect according to the third aspect of the invention include the following:
Zinc pyrithione with tea tree oil;
Zinc pyrithione with tulsi oil;
Zinc pyrithione with eugenol;
Zinc pyrithione with linalool;
Zinc pyrithione with terpinen-4-ol;
Climbazole with tea tree oil;
Climbazole with alpha-terpineol;
Climbazole with eugenol;
Climbazole with linalool;
Climbazole with terpinen-4-ol; and
Octopirox with eugenol.

Of these combinations, those most preferred on account of their greater effectiveness are:
Zinc pyrithione with tulsi oil;
Climbazole with linalool; and
Climbazole with terpinen-4-ol.

Preferably, the compound and the synthetic antimicrobial agent exhibit a Fractional Inhibitory Concentration (FIC) of 0.5 or less. FIC values are for activity against *M.furfur*, such as CBS 7019, and are calculated in ways well-known in the art.

Compositions of the invention may be rinse off products or leave on products. Rinse off products are intended to be substantially rinsed off the hair and/or the scalp of the user with water after use. Leave on products are intended not to be rinsed off the hair and/or the scalp of the user immediately after use (ie, within at least the first 2 hours, preferably at least four hours, after application of the composition). Leave on products include, for example, lotions, creams and hair oils that are intended for topical application to the hair and/or the scalp. Rinse off compositions include shampoos and hair conditioners, as well as hair treatment products which are intended to be left on the hair for up to 2 hours (eg, 5 minutes to 2 hours) before being rinsed off.

A particularly preferred product form is a shampoo. Shampoo compositions preferably comprise from 1% to 50% by weight of one or more surfactants. Shampoo compositions preferably also comprise from 0.001% to 10% of a cationic deposition polymer.

Shampoo compositions of the invention will comprise one or more cleansing surfactants which are cosmetically acceptable and suitable for topical application to the hair. Further surfactants may be present as an additional ingredient if sufficient for cleansing purposes is not provided as emulsifier for any emulsified components in the composition, e.g. emulsified silicones. It is preferred that shampoo compositions of the invention comprise at least one further surfactant (in addition to that used as emulsifying agent) to provide a cleansing benefit.

Suitable cleansing surfactants, which may be used singularly or in combination, are selected from anionic, amphoteric and zwitterionic surfactants, and mixtures thereof. The cleansing surfactant may be the same surfactant as the emulsifier, or may be different.

Examples of anionic surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, and alpha-olefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Typical anionic surfactants for use in shampoos of the invention include sodium oleyl succinate, ammonium lauryl sulphosuccinate, ammonium lauryl sulphate, sodium dodecylbenzene sulphonate, triethanolamine dodecylbenzene sulphonate, sodium cocoyl isethionate, sodium lauryl isethionate and sodium N-lauryl sarcosinate. The most preferred anionic surfactants are sodium lauryl sulphate, triethanolamine monolauryl phosphate, sodium lauryl ether sulphate 1 EO, 2EO and 3EO, ammonium lauryl sulphate and ammonium lauryl ether sulphate 1EO, 2EO and 3EO.

Examples of amphoteric and zwitterionic surfactants include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine and preferably lauryl betaine, cocamidopropyl betaine and sodium cocamphopropionate.

The shampoo compositions can also include co-surfactants, to help impart aesthetic, physical or cleansing properties to the composition. A preferred example is a nonionic surfactant, which can be included in an amount ranging from 0% to about 5% by weight of the total composition.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionics include mono- or di-alkyl alkanolamides. Examples include coco mono- or diethanolamide and coco mono-isopropanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO- (G)ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies in the range of from about 1.1 to about 2. Most preferably the value of n lies in the range of from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

The total amount of surfactant (including any co-surfactant, and/or any emulsifier) in shampoo compositions of the invention is generally from 1 to 50% by weight, preferably from 5 to 30%, more preferably from 10% to 25% by weight of the total shampoo composition.

A cationic deposition polymer is a preferred ingredient in shampoo compositions of the invention, for example for enhancing conditioning performance of the shampoo. By "deposition polymer" is meant an agent which enhances deposition of the silicone component from the shampoo composition onto the intended site during use, i.e. the hair and/or the scalp.

The deposition polymer may be a homopolymer or be formed from two or more types of monomers. The molecular weight of the polymer (in g/mol) will generally be between 5 000 and 10 000 000, typically at least 10 000 and preferably in the range 100 000 to about 2 000 000. The polymers will have cationic nitrogen containing groups such as quaternary ammonium or protonated amino groups, or a mixture thereof.

The cationic nitrogen-containing group will generally be present as a substituent on a fraction of the total monomer units of the deposition polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give a polymer having a cationic charge density in the required range.

Suitable cationic deposition polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic deposition polymers can comprise mixtures of monomer units derived from amine- and/or quaternary ammonium-substituted monomer and/or compatible spacer monomers.

Suitable cationic deposition polymers include, for example:
- copolymers of 1-vinyl-2-pyrrolidine and 1-vinyl-3-methylimidazolium salt (e.g. chloride salt), referred to in the industry by the Cosmetic, Toiletry, and Fragrance Association, (CTFA) as Polyquaternium-16. This material is commercially available from BASF Wyandotte Corp. (Parsippany, NJ, USA) under the LUVIQUAT tradename (e.g. LUVIQUAT FC 370);
- copolymers of 1-vinyl-2-pyrrolidine and dimethylaminoethyl methacrylate, referred to in the industry (CTFA) as Polyquaternium-11. This material is available commercially from Gaf Corporation (Wayne, NJ, USA) under the GAFQUAT tradename (e.g., GAFQUAT 755N);
- cationic diallyl quaternary ammonium-containing polymers including, for example, dimethyldiallyammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and copolymers of unsaturated carboxylic acids having from 3 to 5 carbon atoms, (as described in U.S. Patent 4,009,256);
- cationic polyacrylamides(as described in WO95/22311).

Other cationic deposition polymers that can be used include cationic polysaccharide polymers, such as cationic cellulose derivatives, cationic starch derivatives, and cationic guar gum derivatives.

Cationic polysaccharide polymers suitable for use in compositions of the invention include those of the formula:

A-O-[R-N⁺(R¹) (R²) (R³) X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimonium chloride (Commercially available from Rhodia (formerly Rhone-Poulenc) in their JAGUAR trademark series).

Examples are JAGUAR C13S, which has a low degree of substitution of the cationic groups and high viscosity. JAGUAR C15, having a moderate degree of substitution and a low viscosity, JAGUAR C17 (high degree of substitution, high viscosity), JAGUAR C16, which is a hydroxypropylated cationic guar derivative containing a low level of substituent groups as well as cationic quaternary ammonium groups, and JAGUAR 162 which is a high transparency, medium viscosity guar having a low degree of substitution.

Preferably the cationic deposition polymer is selected from cationic cellulose and cationic guar derivatives. Particularly preferred deposition polymers are JAGUAR C13S, JAGUAR C15, JAGUAR C17 and JAGUAR C16 and JAGUAR C162.

The cationic deposition polymer will generally be present at levels of from 0.001 to 10%, preferably from about 0.01 to 5%, more preferably from about 0.01 to 1%, even more preferably about 0.02% to about 0.5% by weight of the total composition.

Suitable solid active agents that may be incorporated into compositions of the invention include pigment particles, such as solid dyes or colorants suitable for application to hair, and metal colloids.

Hair treatment compositions such as shampoos and conditioners are frequently opacified or pearlised to enhance consumer appeal.

Examples of opacifying agents include higher fatty alcohols (e.g. cetyl, stearyl, arachidyl and behenyl), solid esters (e.g. cetyl palmitate, glyceryl laurate, stearamide MEA-stearate), high molecular weight fatty amides and alkanolamides and various fatty acid derivatives such as propylene glycol and polyethylene glycol esters. Inorganic materials used to opacify hair treatment compositions include magnesium aluminium silicate, zinc oxide, and titanium dioxide.

Pearlescing agents typically form thin, platelet-type crystals in the composition, which act like tiny mirrors. This gives the pearl lustre effect. Some of the opacifying agents listed above may also crystallise as pearlescing agents, depending on the media in which they are used and the conditions employed.

Typical pearlescing agents may be selected from C16-C22 fatty acids (e.g. stearic acid, myristic acid, oleic acid and behenic acid), esters of C16-C22 fatty acid with alcohols and esters of C16-C22 fatty acid incorporating such elements as alkylene glycol units. Suitable alkylene glycol units may include ethylene glycol and propylene glycol. However, higher alkylene chain length glycols may be employed. Suitable higher alkylene chain length glycols include polyethylene glycol and polypropylene glycol.

Examples are polyethylene glycol mono or diesters of C16-C22 fatty acids having from 1 to 7 ethylene oxide units, and ethylene glycol esters of C16-C22 fatty acids. Preferred esters include polyethylene glycol distearates and ethylene glycol distearates. Examples of a polyethylene glycol distearate available commercially are EUPERLAN PK900 (ex Henkel) or GENAPOL TS (ex Hoechst). An example of an ethylene glycol distearate is EUPERLAN PK3000 (ex Henkel).

Other pearlescing agents include alkanolamides of fatty acids having from 16 to 22 carbon atoms, (e.g. stearic monoethanolamide, stearic diethanolamide, stearic monoisopropanolamide and stearic monoethanolamide stearate); long chain esters of long chain fatty acids (e.g. stearyl stearate, cetyl palmitate); glyceryl esters (e.g. glyceryl distearate),long chain esters of long chain alkanolamides (e.g. stearamide DEA distearate, stearamide MEA stearate), and alkyl (C18-C22) dimethyl amine oxides (e.g. stearyl dimethyl amine oxide).

Further suitable pearlescing agents include inorganic materials such as nacreous pigments based on the natural mineral mica. An example is titanium dioxide coated mica. Particles of this material may vary in size from 2 to 150 microns in diameter. In general, smaller particles give rise to a pearly appearance, whereas particles having a larger average diameter will result in a glittery composition.

Suitable titanium dioxide coated mica particles are those sold under the trade names TIMIRON (merck) or FLAMENCO (Mearl).

The level of opacifying or pearlescing agent employed in compositions of the invention is generally from 0.01 to 20%, preferably 0.01 to 5%, more preferably from 0.02 to 2% by weight of the total composition.

Gas (e.g. air) bubbles represent another type of suspended phase that may be introduced into a hair treatment composition for aesthetic purposes. When evenly sized and homogeneously dispersed in the composition, these can enhance consumer appeal - a typical application is in a transparent or translucent composition such as a hair styling gel.

Compositions suitable for the use and the method of the invention may be the shampoo compositions of the invention described above. Suitable compositions may also be formulated as conditioners for the treatment of hair (typically after shampooing) and subsequent rinsing.

Such a conditioner will comprise one or more conditioning surfactants which are cosmetically acceptable and suitable for topical application to the hair. Suitable conditioning surfactants are selected from cationic surfactants, used singly or in admixture. Examples include quaternary ammonium hydroxides or salts thereof, e.g chlorides.

Suitable cationic surfactants for use in hair conditioners of the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in hair conditioners of the invention is cetyltrimethylammonium chloride, available commercially, for example as GENAMIN CTAC, ex Hoechst Celanese.

In conditioners of the invention, the level of cationic surfactant is preferably from 0.01 to 10%, more preferably 0.05 to 5%, most preferably 0.1 to 2% by weight of the composition.

Conditioners of the invention advantageously incorporate a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 20. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention is conveniently from 0.01 to 10%, preferably from 0.1 to 5% by weight of the composition. The weight ratio of cationic surfactant to fatty alcohol is suitably from 10:1 to 1:10, preferably from 4:1 to 1:8, optimally from 1:1 to 1:4.

The compositions which may be used in the invention may contain a conditioning agent. As used herein, the term "conditioning agent" includes any material which is used to give a particular conditioning benefit to hair and/or skin. For example, in compositions for use in washing hair, such as shampoos and conditioners, suitable materials are those which deliver one or more benefits relating to shine, softness, combability, wet-handling, anti-static properties, protection against damage, body, volume, stylability and manageability.

Preferred conditioning agents for use in the present invention include emulsified silicones, used to impart for example wet and dry conditioning benefits to hair such as softness, smooth feel and ease of combability.

Various methods of making emulsions of particles of silicones for use in the invention are available and are well known and documented in the art.

The viscosity of the silicone itself (not the emulsion or the final washing composition) preferably ranges from 10,000 cps to 5 million cps. The viscosity can be measured by means of a glass capillary viscometer as set out further in Dow Corning Corporate Test Method CTM004 July 20 1970.

Suitable silicones include polydiorganosiloxanes, in particular polydimethylsiloxanes which have the CTFA designation dimethicone. An example is dimethicone fluid having a viscosity of up to 100,000 centistokes at 25° C, which is available commercially from the General Electric Company as the Viscasil series and from Dow Corning as the DC 200 series.

Aminofunctional silicones which have the CTFA designation amodimethicone, are also suitable for use in the compositions of the invention, as are polydimethyl siloxanes having hydroxyl end groups, which have the CTFA designation dimethiconol.

Also suitable are silicone gums. "Silicone gum" denotes polydiorganosiloxanes having a molecular weight of from 200,000 to 1,000,000 and specific examples include dimethicone gums, dimethiconol gums, polydimethyl siloxane/diphenyl/methylvinylsiloxane copolymers, polydimethylsiloxane/methylvinylsiloxane copolymers and mixtures thereof. Examples include those materials described in US Pat. No. 4,152,416 (Spitzer), and on General Electric Silicone Rubber product Data Sheet SE 30, SE 33, SE 54 and SE 76.

Also suitable for use in the present invention are silicone gums having a slight degree of cross-linking, as are described for example in WO 96/31188. These materials can impart body, volume and stylability to hair, as well as good wet and dry conditioning.

Preferred emulsified silicones for use in compositions of the invention have an average silicone particle size in the composition of less than 100, preferably less than 30, more preferably less than 20 microns, most preferably less than 10 microns.

Particle size may be measured by means of a laser light scattering technique, using a 2600D Particle Sizer from Malvern Instruments.

Suitable silicone emulsions for use in the invention are commercially available in a pre-emulsified form. This is particularly preferred since the pre-formed emulsion can be incorporated into the washing composition by simple mixing.

Examples of suitable pre-formed emulsions include emulsions DC2-1766 and DC2-1784, available from Dow Corning. These are emulsions of dimethiconol. Cross-linked silicone gums are also available in a pre-emulsified form, which is advantageous for ease of formulation. A preferred example is the material available from Dow Corning as DC X2-1787, which is an emulsion of cross-linked dimethiconol gum.

The amount of silicone incorporated into the compositions of the invention depends on the level of conditioning desired and the material used. A preferred amount is from 0.01 to about 10% by weight of the total composition although these limits are not absolute. The lower limit is determined by the minimum level to achieve conditioning and the upper limit by the maximum level to avoid making the hair and/or skin unacceptably greasy. We have found that an amount of silicone of from 0.5 to 1.5% by weight of the total composition, is a particularly suitable level.

A further preferred class of conditioning agents are peralk(en)yl hydrocarbon materials, used to enhance the body, volume and stylability of hair.

EP 567 326 and EP 498 119 describe suitable peralk(en)yl hydrocarbon materials for imparting stylability and enhanced body to hair. Preferred materials are polyisobutylene materials available from Presperse, Inc. under the PERMETHYL trade name.

The amount of per-alk(en)yl hydrocarbon material incorporated into the compositions of the invention depends on the level of body and volume enhancement desired and the specific material used. A preferred amount is from 0.01 to about 10% by weight of the total composition although these limits are not absolute. The lower limit is determined by the minimum level to achieve the body and volume enhancing effect and the upper limit by the maximum level to avoid making the hair unacceptably stiff. We have found that an amount of per-alk(en)yl hydrocarbon material of from 0.5 to 2% by weight of the total composition is a particularly suitable level.

Compositions of this invention may contain any other ingredient normally used in hair treatment formulations. These other ingredients may include viscosity modifiers, preservatives, colouring agents, polyols such as glycerine and polypropylene glycol, chelating agents such as EDTA, antioxidants, fragrances, and sunscreens. Each of these ingredients will be present in an amount effective to accomplish its purpose. Generally these optional ingredients are included individually at a level of up to about 5% by weight of the total composition.

Preferably, compositions of this invention also contain adjuvants suitable for hair care. Generally such ingredients are included individually at a level of up to 2%, preferably up to 1%, by weight of the total composition.

Among suitable hair care adjuvants, are:
(i) natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts. A particularly preferred combination of natural hair root nutrients for inclusion in compositions of the invention is isoleucine and glucose. A particularly preferred amino acid nutrient is arginine.
(ii) hair fibre benefit agents. Examples are:
   - ceramides, for moisturising the fibre and maintaining cuticle integrity. Ceramides are available by extraction from natural sources, or as synthetic ceramides and pseudoceramides. A preferred ceramide is Ceramide II, ex Quest. Mixtures of ceramides may also be suitable, such as Ceramides LS, ex Laboratoires Serobiologiques. The ceramides may constitute all or part of the lipophilic agent of the invention.

The invention will now be further illustrated by the following, non-limiting examples. In the examples and throughout this specification, all percentages given in relation to formulations are percentages by weight based on total composition and on active ingredient unless otherwise indicated.

### Examples

Studies into anti-microbial action were performed using a microtitre plate cross-assay in triplicate against the type strain *Malassezia furfur* CBS 7019. The Fractional Inhibitory Concentration (FIC), the concentration of biocide in a mixture of biocides that inhibits growth, was determined for natural oils and a number of terpenoid components with the conventional anti-dandruff/anti-fungal agents. The results were as follows.

**MIC values for oils and their components:**

| | |
|---|---|
| Tulsi oil | 0.25% |
| Tea Tree Oil | 0.03 - 0.12% |
| α-terpineol | 0.02% |
| Limonene | >8% |
| Camphene | >8% |
| 4-allylanisole | 0.25% |
| Cineole | 1% |
| Linalool | 0.01% |
| Eugenol | 0.01% |
| α-pinene | 0.3% |

### Example 1

**FIC values with climbazole:**

| | | |
|---|---|---|
| Tea Tree oil | 0.833 | synergy |
| Tulsi oil | 2 | antagonistic |
| α-terpineol | 0.625 | synergy |
| eugenol | 0.56 | synergy |
| linalool | 0.417 | synergy |
| limonene | 2 | antagonistic |
| α-pinene | 2 | antagonistic |
| 4-allylanisole | 2 | antagonistic |
| terpinen-4-ol | 0.37 | synergy |

### Example 2

**FIC values with zinc pyrithione (ZnPTO):**

| | | |
|---|---|---|
| Tea Tree oil | 0.75 | synergy |
| Tulsi oil | 0.45 | synergy |
| α-terpineol | 2 | antagonistic |
| eugenol | 0.625 | synergy |
| linalool | 0.625 | synergy |
| limonene | 1.5 | antagonistic |
| α-pinene | 1.42 | antagonistic |
| 4-allylanisole | 0.725 | synergy |
| Terpinen-4-ol | 0.83 | synergy |

### Example 3

**FIC values with Octopirox:**

| | | |
|---|---|---|
| Tea Tree oil | 2 | |
| Tulsi oil | 2 | |
| α-terpineol | 2 | |
| eugenol | 0.567 | synergy |
| linalool | 2 | antagonistic |
| limonene | 2 | antagonistic |
| α-pinene | 2 | antagonistic |
| 4-allylanisole | 2 | antagonistic |
| Terpinen-4-ol | 2 | antagonistic |

The definition of synergy is a combination of biocides in which the effect is greater than the sum of the expected effects of the individual components.

| | |
|---|---|
| <1 | synergy |
| =1 | additive effect |
| >1 | antagonistic |
| <0.5 | strong synergy |

Strong synergy is therefore exhibited between tulsi oil and zinc pyrithione. Strong synergy is also shown by the combinations of linalool and terpinen-4-ol with climbazole. Surprisingly, eugenol exhibited synergy with the variety of different synthetic antimicrobial agents tested.

### Example 4

The following is an example of a shampoo formulation according to the invention. Percentages are by weight of active material based on total formulation.

| **Chemical Name** | **Supplier** | **Trade name** | **Example 4 (%)** |
|---|---|---|---|
| SLES 2 EO | Albright and Wilson | Empicol ESB 28 | 16.0 |
| CAPB | Goldschmidt | Tegobetaine | 2.0 |
| GUAR HYDROXYPROPYLTRIMONIUM CHLORIDE | Rhone Poulenc | Jaguar C 17 | 0.2 |
| DMDM HYDANTION | Lonza | Glydant LTD | 0.3 |
| Zinc pyrithione | Olin Chemicals | Zinc Omadine | 0.5 |
| Ethanol | Fisher | Ethanol | 1.5 |
| PERFUME | Firmenich | Trouble shooter (perfume) UN 116416 | 0.6 |
| Tulsi oil | Alban-Muller International | | 0.1 |
| NaCl | Fisher | Salt | 0.0 - 1.0 |
| WATER | Local | WATER | To 100 % |

## Claims

1. Hair and/or scalp treatment composition comprising tulsi oil and a metal pyrithione, wherein the tulsi oil and the metal pyrithione exhibit a synergistic antimicrobial activity.

2. Composition as claimed in Claim 1, wherein the tulsi oil is present in the composition in an amount of from 0.0001% to 5% by weight.

3. Composition as claimed in Claim 1 or Claim 2, wherein the metal pyrithione is present in the composition in an amount of from 0.01% to 5% by weight.

4. Composition as claimed in any one of the preceding claims, wherein the weight ratio of the amount of tulsi oil to the amount of metal pyrithione lies within the range of from 1:2 to 1:50.

5. Composition as claimed in any one of the preceding claims, wherein the metal pyrithione is zinc pyrithione.

6. Composition as claimed in any one of the preceding claims, wherein the tulsi oil and the metal pyrithione exhibit a Fractional Inhibitory Concentration of 0.5 or less.

7. Composition as claimed in any one of the preceding claims, which is a rinse off product.

8. Composition as claimed in any one of Claims 1 to 6, which is a leave on product.

9. Composition as claimed in Claim 8, which is a lotion, a cream or a hair oil.

10. Composition as claimed in Claim 7 which is a shampoo or hair conditioner.

11. Composition as claimed in Claim 10, which is a shampoo comprising from 1% to 50% by weight of one or more surfactants.

12. Composition as claimed in Claim 11 which comprises from 0.001% to 10% by weight of a cationic deposition polymer.

## Patentansprüche

1. Haar- und/oder Kopfhautbehandlungszusammensetzung, umfassend Tulsiöl und ein Metallpyrithion, wobei das Tulsiöl und das Metallpyrithion eine synergistische antimikrobielle Wirksamkeit zeigen.

2. Zusammensetzung nach Anspruch 1, wobei das Tulsiöl in der Zusammensetzung in einer Menge von 0,0001 % bis 5 Gewichtsprozent vorliegt.

3. Zusammensetzung nach Anspruch 1 oder Anspruch 2, wobei das Metallpyrithion in der Zusammensetzung in einer Menge von 0,01 % bis 5 Gewichtsprozent vorliegt.

4. Zusammensetzung nach einem der vorangehenden Ansprüche, wobei das Gewichtsverhältnis von der Menge an Tulsiöl zu der Menge an Metallpyrithion im Bereich von 1 : 2 bis 1 : 50 liegt.

5. Zusammensetzung nach einem der vorangehenden Ansprüche, wobei das Metallpyrithion Zinkpyrithion ist.

6. Zusammensetzung nach einem der vorangehenden Ansprüche, wobei das Tulsiöl und das Metallpyrithion eine fraktionelle Inhibitor-Konzentration (FIC) von 0,5 oder weniger zeigen.

7. Zusammensetzung nach einem der vorangehenden Ansprüche, die ein Abspülprodukt ist.

8. Zusammensetzung nach einem der Ansprüche 1 bis 6, die ein Leave-on-Produkt ist.

9. Zusammensetzung nach Anspruch 8, die eine Lotion, eine Creme oder ein Haaröl ist.

10. Zusammensetzung nach Anspruch 7, die ein Shampoo oder ein Haarkonditionierer ist.

11. Zusammensetzung nach Anspruch 10, die ein Shampoo, umfassend 1 % bis 50 Gewichtsprozent von einem oder mehreren Tensiden ist.

12. Zusammensetzung nach Anspruch 11, die 0,001 % bis 10 Gewichtsprozent von einem kationischen Abscheidungspolymer umfasst.

## Revendications

1. Composition pour le traitement des cheveux et/ou du cuir chevelu comprenant de l'huile de tulasi et de la pyrithione métallique, dans laquelle l'huile de tulasi et la pyrithione métallique font preuve d'une activité antimicrobienne synergique.

2. Composition selon la Revendication 1, dans laquelle l'huile de tulasi est présente dans la composition dans une quantité de 0,0001 % à 5 % en poids.

3. Composition selon la Revendication 1 ou la Revendication 2, dans laquelle la pyrithione de zinc est présente dans la composition dans une quantité de 0,01 % à 5 % en poids.

4. Composition selon l'une quelconque des revendications précédentes, dans laquelle le rapport pondéral entre la quantité d'huile de tulasi et la quantité de pyrithione métallique s'établit dans la plage allant de 1 :2 à 1 :50.

5. Composition selon l'une quelconque des revendications précédentes, dans laquelle la pyrithione métallique est de la pyrithione de zinc.

6. Composition selon l'une quelconque des revendications précédentes, dans laquelle l'huile de tulasi et la pyrithione métallique ont une Concentration Inhibitrice Fractionnée de 0,5 ou moins.

7. Composition selon l'une quelconque des revendications précédentes, qui est un produit à rincer.

8. Composition selon l'une quelconque des revendications 1 à 6, qui est un produit sans rinçage.

9. Composition selon la revendication 8, qui est une lotion, une crème ou une huile capillaire.

10. Composition selon la revendication 7, qui est un shampoing ou un conditionneur pour cheveux.

11. Composition selon la revendication 10, qui est un shampoing comprenant de 1 % à 50 % en poids d'un ou de plusieurs tensioactifs.

12. Composition selon la revendication 11, qui comprend de 0,001 % à 10 % en poids d'un polymère de dépôt cationique.
